# EUROPÄISCHE PATENTSCHRIFT

(11) **EP 1 284 255 B1**
(45) Veröffentlichungstag und Bekanntmachung des Hinweises auf die Patenterteilung: **02.11.2006**
(21) Anmeldenummer: 02016938.9
(22) Anmeldetag: 01.08.2002
(51) Int. Cl.: C07C 67/00, C07C 67/343, C07C 69/734, C07B 37/04

(54) **Verfahren zur Arylierung von Olefinen**
Process for the arylation of olefines
Procédé pour l'arylation d'oléfines

(30) Priorität: 13.08.2001 DE 10139722
(43) Veröffentlichungstag der Anmeldung: 19.02.2003
(73) Patentinhaber: Saltigo GmbH, 51369 Leverkusen (DE)
(72) Erfinder: Eckert, Markus, 50937 Köln (DE); Schnatterer, Albert, 51373 Leverkusen (DE); Lange, Walter, 51519 Odenthal (DE)
(74) Vertreter: Wichmann, Birgid

(56) Entgegenhaltungen:
- EP-A- 0 553 668
- DE-A- 4 111 620
- DE-A- 4 111 657
- US-A- 5 231 223
- US-A- 5 300 675
- KIKUKAWA ET AL: "Palladium(0)-Catalyzed Arylation of Olefins by Arylamines and an Alkyl Nitrite" J.ORG.CHEM., Bd. 46, 1981, Seiten 4885-4888, XP000882385

## Beschreibung

Die Erfindung betrifft ein Verfahren zur Arylierung von Olefinen durch Umsetzung von aromatischen Diazoniumsalzen mit Olefinen in Gegenwart eines PalladiumKatalysators.

Arylolefine spielen sowohl als Wirkstoffe oder Wirkstoffzwischenprodukte in Lichtschutzmitteln und Arzneimitteln als auch bei der Herstellung von Farbstoffen eine wichtige Rolle. Die Palladium-katalysierte Umsetzung von Diazoniumsalzen mit Olefinen ist zum Beispiel aus R. F. Heck, Palladium Reagents in Organic Synthesis, Academic Press, 1985, p. 287-290 bekannt. Auch in EP-A 508 264 wird ein Verfahren beschrieben, das diese Reaktion zur Synthese von Arylolefinen ausnutzt. Der Nachteil dieses Verfahrens liegt jedoch in der Tatsache, dass zunächst aus substituierten Anilinen die entsprechenden Diazoniumsalze hergestellt werden und erst anschließend die Umsetzung mit Olefinen unter Zusatz von Palladium-Verbindungen erfolgt. Diese Vorgehensweise besitzt für den industriellen Maßstab den Nachteil, dass zwischenzeitlich große Mengen an Diazoniumsalzen vorliegen, die sowohl ein erhebliches Gefahrenpotential als auch wegen ihrer Schwerlöslichkeit häufig prozesstechnische Probleme mit sich bringen.

Auch das Verfahren aus EP-A 606 057, das als Ausgangsprodukt isolierte Diazoniumsalze benötigt, besitzt oben genannte Nachteile, da immer von der Umsetzung eines bereits gebildeten Diazoniumsalzes in Gegenwart von Ethylen und einem Palladiumkatalysator ausgegangen wird.

In EP-A 553 668 wird ein Verfahren zur Herstellung von Arylacrylsäurederivaten beschrieben, wobei zunächst die Diazotierung von substituierten Anilinen erfolgt und anschließend, durch die Verwendung von Carbonsäuren als Lösungsmittel ohne Zwischenisolierung, die weitere Umsetzung zu Arylacrylsäurederivaten. Jedoch wird auch in diesem Prozess eine Akkumulation von Diazoniumsalzen nicht verhindert.

Ebenfalls in der DE-A-14111620 wird von vorhergebildeten Aryldiazoniumsalzen ausgegangen.

Die US-A-5300675 beschreibt ein Verfahren, bei dem von p-Methoxybenzoldiazoniumtetrafluorboraten als eine der Ausgangsverbindungen ausgegangen wird.

Es bestand daher das Bedürfnis, ein Verfahren zu entwickeln, das ausgehend von gegebenenfalls substituierten Aminoaryl-Verbindungen die Palladium-katalysierte Herstellung von Arylolefinen erlaubt, wobei das intermediär gebildete DiazoniumSalz in situ weiter umgesetzt wird.

Es wurde nun ein Verfahren zur Herstellung von Arylolefinen gefunden, das dadurch gekennzeichnet ist, dass
- ein oder mehrere Arylamine
- in Gegenwart ein oder mehrerer Olefine, die an der Doppelbindung mindestens ein Wasserstoff-Atom tragen und
- in Gegenwart von Säure und
- in Gegenwart von Palladium oder einer oder mehrerer Palladium-Verbindungen oder Palladium und einer oder mehrerer Palladium-Verbindungen
- mit einem oder mehreren organischen Nitriten
umgesetzt werden, wobei das Olefin das Arylamin, die Säure und der Palladiumkatalysator vorgelegt werden und das organische Nitrit zudosiert wird.

Bevorzugt wird das erfindungsgemäße Verfahren so durchgeführt, dass jeweils ein Arylamin ein Olefin und Palladium oder eine Palladium-Verbindung sowie ein organisches Nitrit eingesetzt wird.

Als Arylamine können beispielsweise carbocyclische aromatische oder heteroaromatische Amine eingesetzt werden.

Arylamine bedeuten in diesem Zusammenhang beispielsweise solche der allgemeinen Formel (I)

Ar-(NH₂)ₙ (I)

in der
- n: für eins oder zwei und
- Ar: stehen kann für carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe OH, Iod, Brom, Chlor, Fluor, Nitro, Cyano, freies oder geschütztes Formyl, C₁-C₁₂-Alkyl, C₆-C₁₂-Aryl, C₇-C₁₃-Arylalkyl, C₁-C₈₋Hydroxyalkyl, C₁-C₈-Hydroxyalkoxy, C₁-C₈-Hydroxyalkylamino, -PO-[(C₁₋C₈)-Alkyl]₂, -PO-[(C₆-C₁₂)-Aryl]₂, Tri(C₁-C₆-alkyl)siloxyl oder Resten der allgemeinen Formel (II),

A-B-D-E (II)
in der unabhängig voneinander
- A: fehlt oder für einen C₁-C₈-Alkylenrest steht und
- B: fehlt oder für Sauerstoff, Schwefel oder NR¹ steht,
wobei R¹ Wasserstoff, C₁-C₈-Alkyl, C₇-C₁₀-Arylalkyl oder C₆-C₁₀-Aryl bedeutet und
- D: für eine Carbonyl-Gruppe steht und
- E: für R², OR², NHR³ oder N(R³)₂ steht,
wobei R² für C₁-C₈-Alkyl, C₇-C₁₀-Arylalkyl, C₁-C₈-Hydroxyalkyl, C₁-C₈-Halogenalkyl oder C₆-C₁₀-Aryl und
- R³: jeweils unabhängig für C₁-C₈-Alkyl, C₁-C₈-Hydroxyalkyl, C₇-C₁₀-Arylalkyl oder C₆-C₁₀-Aryl oder N(R³)₂ zusammen für einen cyclischen Aminorest steht,
oder Resten der allgemeinen Formeln (IIIa-e)

A-E (IIIa)

A-SO₂-E (IIIb)

A-B-SO₂R² (IIIc)

A-SO₃X (IIId)

A-COX (IIIe)

in denen A, B, E und R² die oben angegebene Bedeutung besitzen und X für OH, NH₂, oder OM steht, wobei M ein Alkalimetallion, ein halbes Äquivalent eines Erdalkalimetallions, ein Ammoniumion oder ein organisches Ammoniumion bedeuten kann.

Für alle Fälle in denen Arylamine der allgemeinen Formel (I) durch Carbon- oder Sulfonsäuregruppen substituiert sind, können sie auch als inneres Salz vorliegen.

Alkyl beziehungsweise Alkylen bedeutet in den oben genannten Zusammenhängen jeweils unabhängig einen geradkettigen, cyclischen, verzweigten oder unverzweigten Alkyl- beziehungsweise Alkylen-Rest. Gleiches gilt für den Alkylteil eines Arylalkyl-Restes.

Die allgemeine Bezeichnung Aryl schließt nicht nur carbocyclische Reste ein sondern umfasst auch heteroaromatische Reste in denen keines, ein, zwei oder drei Gerüstkohlenstoffatome pro Cyclus, im gesamten Rest mindestens jedoch ein Gerüstkohlenstoffatom, durch Heteroatome, ausgewählt aus der Gruppe Stickstoff, Schwefel oder Sauerstoff, substituiert ist.

Gleiches gilt für den Arylteil eines Arylalkyl-Restes.

Halogenalkyl bedeuten in den oben genannten Zusammenhängen jeweils unabhängig geradkettige, cyclische, verzweigte oder unverzweigte Alkylreste, die mit einem, mehreren oder vollständig mit Halogenatomen unabhängig voneinander ausgewählt aus der Gruppe Fluor-, Chlor-, oder Brom substituiert sein können.

Hydroxyalkyl bedeutet in den oben genannten Zusammenhängen jeweils unabhängig geradkettige, cyclische, verzweigte oder unverzweigte Alkylreste, die in der Art mit einer oder mehreren Hydroxygruppen substituiert sind, dass jedes Kohlenstoffatom des Restes höchstens ein Sauerstoff-, Schwefel- oder Stickstoffatom trägt.

Geschütztes Formyl bedeutet einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Für die regiochemische Anordung der genannten Substituenten zur Aminogruppe oder den Aminogruppen bestehen keine Beschränkungen.

Beispiele für carbocyclische aromatische Reste mit 6 bis 18 Gerüstkohlenstoffatomen sind zum Beispiel Phenyl, Naphtyl, Phenanthrenyl, Anthracenyl oder Fluorenyl, heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Thiophen-yl, Benzofuranyl, Benzothiophen-yl, Dibenzofuran-yl, Dibenzothiophen-yl, Furanyl, Indolyl, Pyridazinyl, Pyrazinyl, Pyrimidinyl, Triazolyl oder Chinolinyl.

Beispiele für Verbindungen der allgemeinen Formel (I) sind die isomeren Aminobenzolsulfonsäuren wie 2-, 3- oder 4-Aminobenzolsulfonsäure, die isomeren Diaminobenzolsulfonsäuren wie 2,4-Diaminobenzolsulfonsäure oder 2,5-Diaminobenzolsulfonsäure, die isomeren Aminobenzoldisulfonsäuren wie Aminobenzol-2,4-disulfonsäure, Aminobenzol-3,5-disulfonsäure und Aminobenzol-2,5-disulfonsäure, die isomeren Diaminobenzoldisulfonsäuren wie 1,4-Diaminobenzol-2,6-disulfonsäure oder 1,3-Diaminobenzol-4,6-disulfonsäure, weiterhin 2-, 3- oder 4-Aminobenzoesäure, 2-, 3- oder 4-Aminobenzoesäure-C₁-C₁₂-alkylester, wie 2-, 3- oder 4-Aminobenzoesäuremethylester, 2-, 3- oder 4-Aminobenzoesäureethylester, 2-, 3-oder 4-Aminobenzoesäure-n-propylester, 2-, 3- oder 4-Aminobenzoesäure-i-propylester, 2-, 3- oder 4-Aminobenzoesäure-n-butylester, 2-, 3- oder 4-Aminobenzoesäure-C₆-C₁₆-arylester wie 2-, 3- oder 4-Aminobenzoesäurephenylester, 3- oder 4-Aminobenzol-1,2-dicarbonsäure, 3- oder 4-Aminobenzol-1,2-dicarbonsäuredi-C₁₋C₁₂-alkylester wie 3- oder 4-Aminobenzol-1,2-dicarbonsäuredimethylester oder 3-oder 4-Aminobenzol-1,2-dicarbonsäurediethylester, 3- oder 4-Aminobenzol-1,2-dicarbonsäureanhydrid, 2-, 3- oder 4-Aminobenzonitril, 3- oder 4-Aminophthalodinitril oder Amino-C₁-C₁₂-alkoxybenzole wie 2-, 3- oder 4-Aminomethoxybenzol, 2-, 3- oder 4-Aminoethoxybenzol, 2-, 3- oder 4-Aminotert. butoxybenzol, 2-, 3- oder 4-Aminophenyl-C₆-C₁₉-arylether, 2-, 3- oder 4-Aminonitrobenzol, 2-, 3- oder 4-Aminofluorbenzol, 2-, 3- oder 4-Aminochlorbenzol, 2-, 3-oder 4-Aminobrombenzol, 2-, 3- oder 4-Aminoiodbenzol, die isomeren Aminofluorchlorbenzole wie 3-Amino-2-fluorchlorbenzol, die isomeren Aminobromchlorbenzole, die isomeren Aminofluorbrombenzole, die isomeren Aminodifluorbenzole, die isomeren Aminodichlorbenzole, die isomeren Aminodibrombenzole, die isomeren Aminodiiodbenzole, 2-, 3- oder 4-Amino(trifluormethylbenzol), (2-, 3-oder 4-Aminophenyl)-C₁-C₁₂-alkylketone wie 2-, 3- oder 4-Aminoacetophenon, (2-, 3- oder 4-Aminophenyl)-C₆-C₁₀-Arylketone wie 2-, 3- oder 4-Aminobenzophenon, 2-, 3- oder 4-Amino-C₁-C₁₂-Alkylbenzole wie 2-, 3- oder 4-Aminotoluol, die isomeren 2-, 3- oder 4-Aminodi-C₁-C₁₂-alkylbenzole wie 3- oder 4-Amino-o-xylol, die isomeren Diamino-C₁-C₁₂-alkylbenzole, wie 2,3-Diaminotoluol, 2,4-Diaminotoluol, 2,5-Diaminotoluol oder 2,6-Diaminotoluol, ferner Diaminodi-C₁₋C₁₂-alkylbenzole, 2-, 3- oder 4-Amino-C₆-C₁₀-arylbenzole wie 2-, 3- oder 4-Aminobiphenyl, 2-, 3- oder 4-Aminobenzol, 2-, 3- oder 4-Aminophenol. Weiter seien genannt die isomeren Aminonaphthalinsulfonsäuren wie 8-Amino-2-naphthalinsulfonsäure, 8-Amino-1-naphthalinsulfonsäure, 7-Amino-1-naphthalinsulfonsäure, 6-Amino-2-naphthalinsulfonsäure, 5-Amino-2-naphthalinsulfonsäure, 5-Amino-1-naphthalinsulfonsäure, 4-Amino-2-naphthalinsulfonsäure, 2-Aminonaphthalin-1-sulfonsäure, 1-Amino-2-naphthalinsulfonsäure, die isomeren Aminonaphthalindisulfonsäuren wie 7-Amino-1,3-naphthalindisulfonsäure, 3-Amino-2,6-naphthalindisulfonsäure, 3-Amino-2,7-naphthalindisulfonsäure, 4-Amino-1,3-naphthalindisulfonsäure, 4-Amino-1,5-naphthalindisulfonsäure, 4-Amino-1,6-naphthalindisulfonsäure, 4-Amino-1,7-naphthalindisulfonsäure, 4-Amino-2,6-naphthalindisulfonsäure, 6-Amino-1,3-naphthalindisulfonsäure, 8-Amino-1,3-naphthalindisulfonsäure, 3-Amino-1,5-naphthalindisulfonsäure, 4-Amino-2,7-naphthalindisulfonsäure, 5-Amino-1,3-naphthalindisulfonsäure, die isomeren Diaminonaphthalinsulfonsäuren und Diaminonaphthalindisulfonsäuren wie 3,4-Bis(Amino)-1-naphthalinsulfonsäure, 4,5-Diamino-1-naphthalinsulfonsäure, 3,8-Diamino-1,5-naphthalindisulfonsäure, 4,8-Diamino-2,6-naphthalin-disulfonsäure, 5,6-Diamino-1,3-naphthalindisulfonsäure, 4,5-Diamino-2,7-naphthalin-disulfonsäure, ferner Diaminobiphenyl-mono- und - disulfonsäuren wie 4,4'-Diaminobiphenyl-3-sulfonsäure und 4,4'-Diaminobiphenyl-3,3'-disulfonsäure.

Darüber hinaus seien genannt 2-, 3-, oder 4-Aminopyridin und 2-, 3-, 4-, 5-, 6-, 7-, 8-Aminochinolin.

Bevorzugte Verbindungen der allgemeinen Formel (I) sind solche, in denen in der allgemeinen Formel (I) n gleich eins ist und Ar für Phenyl, Naphtyl, Anthracenyl, Phenanthrenyl, Pyridinyl, Pyrazinyl, Pyridazinyl, oder Pyrimidinyl steht, die pro Cyclus mit keinem, einem, zwei oder drei weiteren Substituenten aus der Reihe OH, Brom, Chlor, Fluor, Cyano, Methyl, Ethyl, n-Propyl, iso-Propyl, n-Butyl, n-Pentyl, n-Hexyl, Phenyl, Benzyl, Trifluormethyl, Pentafluorethyl, Trichlormethyl, -PO-[(C₁₋C₈)-Alkyl]₂, -PO-[(C₆-C₁₂)-Aryl]₂ und Substituenten der allgemeinen Formeln (II) und (IIIa-e), in denen jeweils unabhängig voneinander
- A: fehlt oder für einen Methylen oder 1,2-Ethylen steht
- B: fehlt oder für Sauerstoff oder NR¹ steht,
wobei R¹ Wasserstoff, Methyl, Ethyl oder Propyl bedeutet und
- D: für eine Carbonyl-Gruppe steht und
- E: für R², OR², NHR₃ oder N(R³)₂ steht,
wobei R² für Methyl, Ethyl, n-Propyl, iso-Propyl, n-Butyl, tert.-Butyl, Benzyl, 2-Hydroxyethyl, Trifluormethyl oder Phenyl und
- R³: jeweils unabhängig für Methyl, Ethyl, n-Propyl, iso-Propyl, n-Butyl, Benzyl, 2-Hydroxyethyl oder Phenyl oder
N(R³)₂ zusammen für Morpholinyl, Piperidinyl oder Pyrrolidinyl steht
und X für OH, NH₂, oder OM steht, wobei M für ein Natrium-, Kalium- oder Ammoniumion steht.

Besonders bevorzugte Verbindungen sind solche der allgemeinen Formel (I), in der n gleich eins ist und Ar für Phenyl steht, das mit keinem, einem oder zwei weiteren Substituenten aus der Reihe Fluor, Cyano, Methyl, Ethyl, Phenyl, Trifluormethyl, und solche Reste der allgemeinen Formeln (II) sowie (IIIa,d,e) in denen
A und B fehlen und
- D: für eine Carbonyl-Gruppe steht und
- E: für R², OR², NHR³ oder N(R³)₂ steht,
wobei R² für Methyl, Ethyl oder Phenyl und
- R³: jeweils unabhängig für Methyl, Ethyl oder Phenyl
und X für OH, NH₂, oder ONa steht.

Ganz besonders bevorzugt ist 4-Methoxyaminobenzol.

Als Olefine, die an der Doppelbindung mindestens ein Wasserstoff-Atom tragen, können beispielsweise solche der allgemeinen Formel (V) eingesetzt werden,

R⁵CH=CR⁶R⁷ (V)

in der unabhängig voneinander
- R⁵: für Wasserstoff oder Methyl und
- R⁶: für Wasserstoff oder Methyl und
- R⁷: stehen kann für Wasserstoff, Cyano, SO₃M, C₁-C₈-Alkyl, carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können
oder für Reste der allgemeinen Formel (VI) 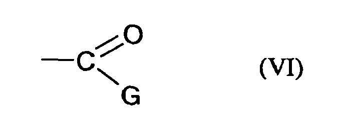 wobei G für OM, OH, NH₂, OR⁸, NHR⁸ oder N(R⁸)² steht und R⁸ für C₁-C₁₂-Alkyl, C₇-C₁₂-Arylalkyl oder C₆-C₁₀-Aryl oder
N(R⁸)₂ zusammen für einen cyclischen Aminorest steht und wobei M für ein Alkalimetallion, ein halbes Äquivalent eines Erdalkalimetallions, ein Ammoniumion oder ein organisches Ammoniumion stehen kann.

Weiterhin können die carbocyclischen aromatischen Reste oder heteroaromatische Reste beispielsweise mit bis zu drei gleichen oder verschiedenen Substituenten pro Cyclus substituiert sein, ausgewählt aus der Gruppe Iod, Brom, Chlor, Fluor, Nitro, Cyano, freies oder geschütztes Formyl, C₁-C₁₂-Alkyl, C₆-C₁₂-Aryl, C₇-C₁₃-Arylalkyl, C₁-C₈-Alkoxy, C₁-C₈-Alkoxycarbonyl, C₁-C₈-Halogenalkoxy, NH(C₁-C₈-Alkyl), N(C₁-C₈-Alkyl)₂.

Beispiele für Olefine der allgemeinen Formel (V) sind Ethen, Propen, Buten, 1,1,1-Trifluor-2-propen, gegebenenfalls substituierte Vinyl-C₆-C₁₀-aromaten wie Styrol oder die isomeren Vinylnaphthaline, 2-, 3- oder 4-Fluorstyrol, 2-, 3- oder 4-Chlorstyrol, 2-, 3- oder 4-Bromstyrol, 2-, 3- oder 4-Iodstyrol, 2-, 3- oder 4-Cyanostyrol, 2-, 3- oder 4-(C₁-C₁₂)-Alkoxystyrol wie 2-, 3- oder 4-Methoxystyrol, 2-, 3- oder 4-Nitrostyrol, 2-, 3- oder 4-Styrolcarbonsäure, 2-, 3- oder 4-Styrolcarbonsäure-C₁-C₁₂ alkylester wie 2-, 3- oder 4-Styrolcarbonsäuremethylester, 2-, 3- oder 4-Styrolcarbonsäure-C₆-C₁₂-Arylester wie 2-, 3- oder 4-Styrolcarbonsäurephenylester, 2-, 3-oder 4-Styrolsulfonsäure bzw. deren Salze, 3- oder 4-Vinylphthalsäure, 3- oder 4-Vinylphthalsäuredi-C₁-C₁₂-alkylester wie 3- oder 4-Vinylphthalsäuredimethylester, 3- oder 4-Vinylphthalsäuredi-C₆-C₁₀-arylester wie 3- oder 4-Vinylphthalsäurediphenylester, 3- oder 4-Vinylphthalsäureanhydrid, Vinylhetaryle wie N-Vinylimidazol oder 2- oder 4-Vinylpyridin, ferner Acrylnitril, Acrylsäure, Acrylsäure-C₁₋C₁₂-alkylester wie Acrylsäuremethylester, Acrylsäureethylester, Acrylsäure-n-propylester, Acrylsäure-2-ethyl-hexylester, Vinylsulfonsäure bzw. deren Salze.

Als Olefine mit mindestens einem Wasserstoff-Substituenten ganz besonders bevorzugt sind Ethylen, Propen, Acrylnitril, Acrylsäure, Acrylsäuremethylester, Acrylsäure(2-ethylhexyl)ester und 1,1,1 -Trifluor-2-propen.

Die Menge des eingesetzten Olefins kann beispielsweise das 0,5 bis 200-fache, bezogen auf die molare Menge der umzusetzenden Amino-Gruppen des Arylamins betragen, das 0,9 bis 5-fache ist bevorzugt, das 1,0 bis 1,2-fache ist ganz besonders bevorzugt.

Als Säure kommen beispielsweise in Frage Schwefelsäure, Halogenwasserstoffsäuren wie Chlorwasserstoff, Bromwasserstoff oder Iodwasserstoff, Phosphorsäure, Essigsäure, Propionsäure, Methansulfonsäure, Trifluormethansulfonsäure, Tetrafluoroborsäure oder Hexafluorophosphorsäure oder Mischungen davon. Die Säuren können auch in Form von wässrigen Lösungen eingesetzt werden.

Bevorzugt kommen konzentrierte Schwefelsäure, wässrige Lösungen von Chlorwasserstoffsäure sowie Essigsäure oder Mischungen davon zum Einsatz, ganz bevorzugt ist konzentrierte Schwefelsäure.

Die Menge der eingesetzten Säure kann beispielsweise das 0,1 bis 10-fache bezogen auf die molare Menge der zu diazotierenden Aminogruppen sein, bevorzugt ist das 0,5 bis 2-fache.

Sind freie Säuregruppen im eingesetzten Arylamin oder dem eingesetzten Olefin vorhanden, kann die Säuremenge auch entsprechend vermindert werden.

Der Einsatz von Palladium im erfindungsgemäßen Verfahren kann beispielsweise in Form von Palladiumschwarz oder auf Trägermaterialien aufgebracht erfolgen, wie zum Beispiel Palladium auf Aktivkohle.

Als Palladium-Verbindungen sind beispielsweise Palladium-Phosphin-Komplexe geeignet, die entweder in situ aus Palladiumsalzen und Phosphin-Liganden hergestellt oder als isolierte Palladium-Phosphin-Komplexe eingesetzt werden.

Als isolierte Palladium-Phosphin-Komplexe können beispielsweise solche der allgemeinen Formel (VIIa) verwendet werden,

[PdL₂An₂] (VIIa)

in der
- L: für jeweils ein Monophosphin oder
- L₂: zusammen für ein Diphosphin und
- An: für das Anion einer Säure steht,
oder solche der allgemeinen Formel (VIIb)

[PdL₄] (VIIb)

in der L jeweils für ein Monophosphin oder ein halbes Äquivalents eines Diphosphins stehen kann.

Monophosphine können beispielsweise solche der allgemeinen Formel (VIII) sein

P(R⁹)₃ (VIII)

in der die Reste R⁹ unabhängig voneinander für geradkettiges, verzweigtes oder cyclisches C₁-C₈-Alkyl oder unsubstituiertes, ein-, zwei oder dreifach durch R¹⁰ substituiertes Phenyl- oder Naphtyl- stehen, wobei
- R¹⁰: für geradkettiges, verzweigtes oder cyclisches C₁-C₈-Alkyl, geradkettiges, verzweigtes oder cyclisches C₁-C₈-Alkoxy, Chlor, Fluor-, N(C₁-C₆-Alkyl)₂, CO₂-(C₁-C₆-Alkyl), -CON(C₁-C₆-Alkyl)₂, Cyano- oder C₁-C₆-Acyl steht.

Bevorzugte Monophosphine sind Triphenylphosphin und Tris-o-tolylphosphin.

Diphosphine können beispielsweise solche der allgemeinen Formel (IX) sein,

(R¹¹)₂P-A-P(R¹¹)₂ (IX)

in der
die Reste R¹¹ unabhängig voneinander für geradkettiges oder cyclisches, verzweigtes oder unverzweigtes C₁-C₈-Alkyl oder für unsubstituiertes, ein-, zwei oder dreifach durch R¹² substituiertes Phenyl-, Naphtyl- oder Heteroaryl mit 5 bis 12 Gerüstkohlenstoffatomen stehen, wobei
- R¹²: jeweils unabhängig ausgewählt ist aus der Gruppe geradkettiges, verzweigtes oder cyclisches C₁-C₈-Alkyl, geradkettiges, verzweigtes oder cyclisches C₁-C₆-Alkoxy, Fluor- oder Cyano- und
- A: für einen unsubstituierten oder substituierten Rest aus der Gruppe C₁-C₄₋Alkylen, 1,2-Phenyl, 1,2-Cyclohexyl, 1,1'-Ferrocenyl, 1,2-Ferrocenyl, 2,2'-(1,1'-Binaphtyl) und 1,1'-Biphenyl steht.

Bevorzugte Diphosphine sind Bis-(Diphenylphosphino)-ethan, 1,3-Bis-(Diphenylphosphino)-propan und 1,4-Bis-(Diphenylphosphino)-butan.

Das Anion einer Säure kann beispielsweise ausgewählt sein aus der Gruppe Chlorid, Bromid, Iodid, Acetat oder Nitrat.

Als Palladiumverbindungen können weiterhin beispielsweise eingesetzt werden Pd₂(dibenzylidenaceton)₃ oder Allylpalladiumchlorid oder -bromid oder solche der allgemeinen Formel (Xa),

Pd(Y¹)₂ (Xa)

in der
- Y¹: für Chlorid, Bromid, Acetat, Nitrat, Methansulfonat, Trifluormethansulfonat oder Acetylacetonat steht,
oder Palladiumverbindungen der allgemeinen Formel (Xb)

Pd(Y²)₂L₂ (Xb)

in der
- Y²: für Chlorid, Bromid, Acetat, Methansulfonat oder Trifluormethansulfonat Tetrafluoroborat oder Hexafluorophosphat steht und
- L: jeweils für Acetonitril, Benzonitril oder Benzylnitril steht, oder
- L₂: zusammen für 1,5-Cyclooctadien steht,
oder Palladiumverbindungen der allgemeinen Formel (Xc)

M₂[Pd(Y³)₄] (Xc),

wobei
- Y³: für Chlorid oder Bromid steht und
- M: für Lithium, Natrium, Kalium, Ammonium oder organisches Ammonium steht.

Bevorzugt sind Palladium auf Kohle und Palladium, Palladiumacetat, Palladiumchlorid, Palladiumbromid, Lithium, Natrium und Kalium-tetrachloropalladat und - tetrabromopalladat, sowie [PdCl₂(triphenylphosphin)₂], [PdBr₂(triphenylphosphin)₂], [PdI₂(triphenylphosphin)₂], [PdCl₂(tris-o-tolylphosphin)₂], [PdBr₂(tris-o-tolylphosphin)₂], [PdI₂(tris-o-tolylphosphin)₂], [PdCl₂(Bis-diphenylphosphinoethan)], [PdBr₂(Bis-diphenylphosphinoethan)], [PdI₂(Bis-diphenylphosphinoethan)], [PdCl₂(1,3-Bis-diphenylphosphinopropan)], [PdBr₂(1,3-Bis-diphenylphosphinopropan)], [PdI₂(1,3-Bis-diphenylphosphinopropan)], [PdCl₂(1,4-Bis-diphenylphosphinobutan)], [PdBr₂(1,4-Bis-diphenylphosphinobutan)], [PdI₂(1,4-Bis-diphenylphosphinobutan)] sowie Palladiumkomplexe, die in situ aus beliebigen Kombinationen von Palladiumacetat, Palladiumnitrat, Palladiumchlorid, Palladiumbromid, Lithium-, Natrium- und Kalium-tetrachloropalladat und -tetrabromopalladat und Triphenylphosphin, Tri-o-tolylphosphin, Bis-diphenylphosphinoethan, 1,3-Bisdiphenylphosphinopropan oder 1,4-Bis-diphenylphosphinobutan erzeugt werden. Weiterhin sind Pd₂(dibenzylidenaceton)₃ und [Pd(triphenylphosphin)₄].

Ganz besonders bevorzugt sind Palladium auf Kohle, Palladiumacetat und Pd₂ (dibenzylidenaceton)₃.

Die Menge des eingesetzten Palladiums oder der eingesetzten Palladiumverbindung kann beispielsweise 0,0001 bis 10 mol-% bezogen auf die eingesetzte Arylverbindung betragen, bevorzugt sind 0,001 bis 5 mol-%, ganz besonders bevorzugt 0,01 bis 2 mol-%.

Als organische Nitrite können beispielsweise solche der allgemeinen Formel (IV) eingesetzt werden,

R⁴ONO (IV)

in der R⁴ beispielsweise für geradkettiges oder verzweigtes, cyclisches oder acyclisches C₁-C₈ Alkyl, oder C₇-C₉-Arylalkyl steht oder Mischungen solcher Nitrite.

Bevorzugt ist der Einsatz nur jeweils eines Nitrits der allgemeinen Formel (IV), in der R für Benzyl, Methyl, Ethyl, n-Propyl, iso-Propyl, n-Butyl, iso-Butyl, tert.-Butyl, n-Pentyl, iso-Pentyl, neo-Pentyl, n-Hexyl oder 2-Ethyl-hexyl steht.

Für das erfindungsgemäße Verfahren ganz besonders bevorzugte organische Nitrite sind Methylnitrit und tert.-Butylnitrit.

Die organischen Nitrite sind entweder kommerziell erhältlich oder dem Fachmann in an sich bekannter Weise herstellbar zum Beispiel durch Umsetzung des zugrundeliegenden Alkohols mit Nitrit-Salzen wie zum Beispiel Natriumnitrit in Gegenwart von Säure.

Die Menge des eingesetzten organischen Nitrits kann beispielsweise zwischen dem 0,8 und 5-fachen bezogen auf die molare Menge der zu diazotierenden Amino-gruppen betragen, bevorzugt ist das 0,9 bis 1,5-fache, ganz besonders bevorzugt ist das 1,0 bis 1,3-fache.

Die Reaktionstemperatur kann beispielsweise 0 bis 150°C, bevorzugt 10 bis 100°C und ganz besonders bevorzugt 50 bis 90°C betragen.

Gegebenenfalls können der Reaktionsmischung auch ein oder mehrere organische Lösungsmittel zugegeben werden.

Als organische Lösungsmittel kommen beispielsweise in Frage:
Alkohole wie Methanol, Ethanol, n-Propanol, iso-Propanol, n-Butanol, iso-Butanol, tert.-Butanol, n-Pentanol, iso-Pentanol, neo-Pentanol, n-Hexanol, cyclo-Hexanol und 2-Ethyl-hexanol,
oder polar aprotische Lösungsmittel wie N-Methylpyrolidon, Dimethylsulfoxid, Tetramethylensulfon, Dimethylformamid, N-Methylcaprolactam, Acetonitril oder Benzonitril.

Ist das zur Reaktion eingesetzte Olefin zumindest in einem Teilbereich der angegebenen Temperaturbereiche flüssig, so kann es auch selbst als Lösungsmittel eingesetzt werden.

Der Druck ist im Allgemeinen unkritisch und kann beispielsweise 0,5 bis 20 bar betragen. Bevorzugt ist Umgebungsdruck. Dadurch ist auch der Einsatz am verflüssigten Olefinen als Lösungsmittel möglich.

Bevorzugt wird die Reaktion unter weitestgehendem Ausschluss von Sauerstoff durchgeführt.

Weiterhin können der Reaktion Verbindungen zugesetzt werden, die als Radikalfänger und/oder Reduktionsmittel dienen. Beispiele für solche Verbindungen sind Hydrochinon, Hydrochinonmonomethylether, 2,6-Di-tert.-butylphenol, 2,6-Di-tert.-butyl-4-methylphenol oder 2-tert.-Butyl-5-methylphenol.

Beim erfindungsgemäßen Verfahren geht man so vor, dass das Olefin, das Arylamin, die Säure, den Palladiumkatalysator sowie gegebenenfalls das Lösungsmittel vorgelegt wird und das organische Nitrit bei der gewünschten Reaktionstemperatur zudosiert. Gegebenfalls kann nach beendeter Zugabe des organischen Nitrits noch bei gleicher Temperatur nachgerührt werden.

In einer Ausführungsform des erfindungsgemäßen Verfahrens setzt man zur Herstellung von speziellen Arylolefinen, den Zimtsäurederivaten, so vor, dass man
- ein oder mehrere Arylamine
- in Gegenwart eines oder mehrerer Olefine der allgemeinen Formel (V), in der R⁵ und R⁶ die weiteste oben genannte Bedeutung besitzen und R⁷ für Reste der allgemeinen Formel (VI) stehen kann, 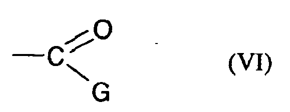
wobei G für OM, OH, OR⁸, und R⁸ für C₁-C₁₂-Alkyl, C₇-C₁₂-Arylalkyl oder C₆-C₁₀-Aryl stehen kann und wobei M für ein Alkalimetallion, ein halbes Äquivalent eines Erdalkalimetallions, ein Ammoniumion oder ein organisches Ammoniumion stehen kann,
- in Gegenwart von Säure und
- in Gegenwart von Palladium oder einer oder mehrere Palladium-Verbindungen oder Palladium und einer oder mehrere Palladium-Verbindungen
- in Gegenwart eines Alkohols der allgemeinen Formel (XI)

   R¹³OH (XI)
- in der R¹³ für C₁-C₁₂-Alkyl oder C₇-C₁₃-Arylalkyl steht
- gegebenenfalls in Gegenwart eines oder mehrerer Lösungsmittel
- mit einem oder mehreren organischen Nitriten
umsetzt.

Bevorzugt wird jeweils ein Arylamin, ein Olefin und ein organisches Nitrit eingesetzt.

In diesem Fall kann die Menge des eingesetzten Alkohols der allgemeinen Formel (XI) das 0,5 bis 200-fache bezogen auf die molare Menge umzusetzender Amino-gruppen des Arylamins betragen, bevorzugt ist das 0,8 bis 50-fache, besonders bevorzugt das 1,0 bis 10-fache.

In einer weiteren Variante zur Herstellung von substituierten Zimtsäuren kann sich das eingesetzte organische Nitrit vorteilhafterweise von dem eingesetzten Alkohol der allgemeinen Formel (XI) ableiten.

Die herstellten Arylolefine eignen sich insbesondere beispielsweise zur Herstellung von Arzneimitteln oder deren Zwischenprodukten und zur Herstellung von Lichtschutzmitteln, besonders UV-A und UV-B-Lichtschutzfiltern, oder deren Zwischenprodukten.

Weiterhin sind die herstellten Arylolefine zur Herstellung von Farbstoffpräparationen oder deren Zwischenprodukten geeignet.

Der besonderer Vorteil des erfindungsgemäßen Verfahrens liegt in der Tatsache, dass die katalytische Umsetzung des intermediär erzeugten Diazoniumsalzes sofort bei der Bildung einsetzt und daher eine vorherige Isolation der sicherheitstechnisch schwierig zu handhabenden Diazoniumsalze oder deren Akkumulation im Reaktionsmedium verhindert werden kann. Ein weiterer Vorteil des erfindungsgemäßen Verfahrens ist, dass zur Herstellung von substituierten Zimtsäureestern in einer Eintopf-Reaktion sowohl die Veresterung als auch die Arylierung des eingesetzten Acrylsäurederivats erfolgen kann.

### Beispiele

### Beispiel 1

Es wurden 6,2 g 4-Methoxy-aminobenzol (p-Anisidin) in 75 ml 2-Ethylhexanol (Isooctanol) gelöst und mit 2 ml konzentrierter Schwefelsäure versetzt. Unter Stickstoffatmosphäre wurden 0,12 g Palladiumacetat, 0,07g Hydrochinon, 1,3g Triphenylphosphin und 9,4 g 2-Ethylhexylacrylat zugegeben und unter Rühren auf 80°C geheizt. Bei dieser Temperatur wurden in die Suspension innerhalb von 1 h Methylnitrit eingeleitet, welches durch Einleiten von 12 ml 48 %iger Schwefelsäure in eine Lösung von 7 g Natriumnitrit in 4 g Methanol in 21 ml Wasser gebildet wurde. Es wurde noch 1 h nachgerührt. Es wurde heiß abgesaugt und die Rohlösung im Vakuum destilliert. So erhielt man 8,6 g 4-Methoxyzimtsäureisooctylester (60 % d.Th.).

### Beispiel 2

Es wurden 6,2 g 4-Methoxy-aminobenzol (p-Anisidin) in 40 ml NMP gelöst und mit 2 ml konzentrierter Schwefelsäure versetzt. Unter Stickstoffatmosphäre wurden 0,6 g Palladium auf Aktivkohle (5 %ig), 0,07 g Hydrochinon und 9,4 g 2-Ethylhexylacrylat zugegeben und unter Rühren auf 80°C geheizt. Bei dieser Temperatur wurden in die Suspension innerhalb von 1 h 7 ml t-Butylnitrit zugetropft. Es wurde noch 1 h nachgerührt. Es wurde heiß abgesaugt und die Rohlösung im Vakuum destilliert. So erhielt man 11,7 g 4-Methoxyzimtsäureisooctylester (81 % d.Th.).

### Beispiel 3

Es wurden 6,2 g 4-Methoxy-aminobenzol (p-Anisidin) in 40 ml 2-Ethylhexanol (Isooctanol) gelöst und mit 2 ml konzentrierter Schwefelsäure versetzt. Unter Stickstoffatmosphäre wurden 0,6 g Palladium auf Aktivkohle (5 %ig), 0,07 g Hydrochinon und 4,2 ml Acrylsäure zugegeben und unter Rühren auf 75°C geheizt.

Bei dieser Temperatur wurden in die Suspension innerhalb von 1 h 7 ml t-Butylnitrit zugetropft. Es wurde noch 1 h nachgerührt. Es wurde heiß abgesaugt und die Rohlösung im Vakuum destilliert. So erhielt man 7,5 g 4-Methoxyzimtsäureisooctylester (52 % d.Th.).

## Patentansprüche

1. Verfahren zur Herstellung von Arylolefinen durch Palladium-katalysierte Kupplung von Aryldiazoniumsalzen und Olefinen **dadurch gekennzeichnet, dass**
- ein oder mehrere Arylamine
- in Gegenwart ein oder mehrerer Olefine, die an der Doppelbindung mindestens ein Wasserstoff-Atom tragen und
- in Gegenwart von Säure und
- in Gegenwart von Palladium oder einer oder mehrerer Palladium-Verbindungen oder Palladium und einer oder mehrerer Palladium-Verbindungen
- mit einem oder mehreren organischen Nitriten
umgesetzt wird, wobei das Olefin, das Arylamin, die Säure und der Palladiumkatalysator vorgelegt werden und das organische Nitrit zudosiert wird.

2. Verfahren nach Anspruch 1, **dadurch gekennzeichnet, dass** ein Arylamin in Gegenwart eines Olefins, das an der Doppelbindung mindestens ein Wasserstoff-Atom trägt in Gegenwart von Palladium oder einer Palladium-Verbindung mit einem organischen Nitrit umgesetzt wird.

3. Verfahren nach einem oder mehreren der Ansprüche 1 bis 2, **dadurch gekennzeichnet, dass** ein oder mehrere organische Lösungsmittel zugesetzt werden.

4. Verfahren nach einem oder mehreren der Ansprüche 1 bis 3, **dadurch gekennzeichnet, dass** es in Gegenwart eines oder mehrerer Radikalfänger durchgeführt wird.

5. Verfahren nach einem oder mehreren der Ansprüche 1 bis 4, **dadurch gekennzeichnet, dass** es in Gegenwart eines oder mehrerer Reduktionsmittel durchgeführt wird.

6. Verfahren nach einem oder mehreren der Ansprüche 1 bis 5, **dadurch gekennzeichnet, dass** als Arylamine carbocyclische aromatische oder heteroaromatische Amine eingesetzt werden.

7. Verfahren nach einem oder mehreren der Ansprüche 1 bis 6, **dadurch gekennzeichnet, dass** als Arylamine solche der allgemeinen Formel (I) eingesetzt werden
Ar-(NH₂)ₙ (I)
in der
n für eins oder zwei und
Ar für carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert ist, steht und die carbocyclischen aromatischen Reste oder heteroaromatische Reste weiterhin mit bis zu fünf gleichen oder verschiedenen Substituenten pro Cyclus substituiert sind, ausgewählt aus der Gruppe OH, Iod, Brom, Chlor, Fluor, Nitro, Cyano, freies oder geschütztes Formyl, C₁-C₁₂-Alkyl, C₆-C₁₂-Aryl, C₇-C₁₃-Arylalkyl, C₁-C₈₋Hydroxyalkyl, C₁-C₈-Hydroxyalkoxy, C₁-C₈-Hydroxyalkylamino, -PO-[(C₁-C₈)-Alkyl]₂, -PO-[(C₆-C₁₂)-Aryl]₂, Tri(C₁-C₆-alkyl)siloxyl oder Resten der allgemeinen Formel (II),
A-B-D-E (II)
in der unabhängig voneinander
A fehlt oder für einen C₁-C₈-Alkylenrest steht und
B fehlt oder für Sauerstoff, Schwefel oder NR¹ steht,
wobei R¹ Wasserstoff, C₁-C₈-Alkyl, C₇-C₁₀-Arylalkyl oder C₆-C₁₀-Aryl bedeutet und
D für eine Carbonyl-Gruppe steht und
E für R², OR², NHR³ oder N(R³)₂ steht,
wobei R² für C₁-C₈-Alkyl, C₇-C₁₀-Arylalkyl, C₁-C₈-Hydroxyalkyl, C₁-C₈-Halogenalkyl oder C₆-C₁₀-Aryl und
R³ jeweils unabhängig für C₁-C₈-Alkyl, C₁-C₈-Hydroxyalkyl, C₇-C₁₀-Arylalkyl oder C₆₋C₁₀-Aryl oder
N(R³)₂ zusammen für einen cyclischen Aminorest steht
oder Resten der allgemeinen Formeln (IIIa-e)
A-E (IIIa)
A-SO₂-E (IIIb)
A-B-SO_{2R}² (IIIc)
A-SO₃X (IIId)
A-COX (IIIe)
in denen A, B, E und R² die oben angegebene Bedeutung besitzen und X für OH, NH₂, oder OM steht, wobei M ein Alkalimetallion, ein halbes Äquivalent eines Erdalkalimetallions, ein Ammoniumion oder ein organisches Ammoniumion bedeutet.

8. Verfahren nach einem oder mehreren der Ansprüche 1 bis 7, **dadurch gekennzeichnet, dass** als Olefine, die an der Doppelbindung mindestens ein Wasserstoff-Atom tragen solche der allgemeinen Formel (V) eingesetzt werden,
R⁵CH=CR⁶R⁷ (V)
in der unabhängig voneinander
R⁵ für Wasserstoff oder Methyl und
R⁶ für Wasserstoff oder Methyl und
R⁷ für Wasserstoff, Cyano, SO₃M, C₁-C₈-Alkyl, carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert ist, steht
oder für Reste der allgemeinen Formel (VI) 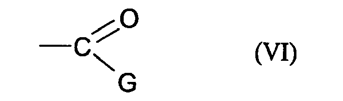 wobei G für OM, OH, NH₂, OR⁸, NHR⁸ oder N(R⁸)² steht und R⁸ für C₁-C₁₂-Alkyl, C₇-C₁₂₋Arylalkyl oder C₆-C₁₀-Aryl oder
N(R8)2 zusammen für einen cyclischen Aminorest steht und wobei M für ein Alkalimetallion, ein halbes Äquivalent eines Erdalkalimetallions, ein Ammoniumion oder ein organisches Ammoniumion steht und
weiterhin die carbocyclischen aromatischen Reste oder heteroaromatische Reste mit bis zu drei gleichen oder verschiedenen Substituenten pro Cyclus substituiert sind, ausgewählt aus der Gruppe Iod, Brom, Chlor, Fluor, Nitro, Cyano, freies oder geschütztes Formyl, C₁-C₁₂₋Alkyl, C₆-C₁₂-Aryl, C₇-C₁₃-Arylalkyl, C₁-C₈-Alkoxy, C₁-C₈-Alkoxycarbonyl, C₁-C₈₋Halogenalkoxy, NH(C₁-C₈-Alkyl), N(C₁-C₈-Alkyl)₂.

9. Verfahren nach einem oder mehreren der Ansprüche 1 bis 8, **dadurch gekennzeichnet, dass** als Säuren Schwefelsäure, Chlorwasserstoffsäure oder Essigsäure eingesetzt werden.

10. Verfahren nach einem oder mehreren der Ansprüche 1 bis 9, **dadurch gekennzeichnet, dass** als organische Nitrite solche der allgemeinen Formel (IV) eingesetzt werden,
R⁴ONO (IV)
in der
R⁴ beispielsweise für geradkettiges oder verzweigtes, cyclisches oder acyclisches C₁₋C₈ Alkyl, oder C₇-C₉-Arylalkyl steht oder Mischungen solcher organischen Nitrite.

11. Verfahren nach einem oder mehreren der Ansprüche 1 bis 7, **dadurch gekennzeichnet, dass** die Menge des eingesetzten Palladiums oder der eingesetzten Palladiumverbindung 0.0001 bis 10 mol-% bezogen auf die eingesetzte Arylverbindung beträgt.

12. Verfahren nach einem oder mehreren der Ansprüche 1 bis 11, **dadurch gekennzeichnet, dass** die Reaktionstemperatur 0 bis 150°C beträgt.

13. Verfahren nach Anspruch 1 zur Herstellung von Zimtsäurederivaten durch Palladium-katalysierte Kupplung von Aryldiazoniumsalzen und Olefinen **dadurch gekennzeichnet, dass**
- ein oder mehrere Arylamine
- in Gegenwart eines oder mehrere Olefine der allgemeinen Formel (V), in der
R⁵ für Wasserstoff oder Methyl und
R⁶ für Wasserstoff oder Methyl und
R⁷ für Reste der allgemeinen Formel (VI) steht, 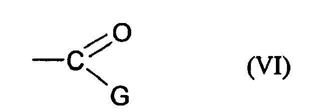 wobei G für OM, OH, OR⁸, und R⁸ für C₁-C₁₂-Alkyl, C₇-C₁₂-Arylalkyl oder C₆₋C₁₀-Aryl stehen kann und wobei M für ein Alkalimetallion, ein halbes Äquivalent eines Erdalkalimetallions, ein Ammoniumion oder ein organisches Ammoniumion steht und
- in Gegenwart von Säure und
- in Gegenwart von Palladium oder einer oder mehrerer Palladium-Verbindungen oder Palladium und einer oder mehrerer Palladium-Verbindungen
- in Gegenwart eines Alkohols der allgemeinen Formel (XI)
R¹³OH (XI)
- in der R¹³ für C₁-C₁₂-Alkyl oder C₇-C₁₃-Arylalkyl steht
- mit einem oder mehreren organischen Nitriten umgesetzt wird, wobei das Olefin, das Arylamin, die Säure und der Palladium Katalysator vorgelegt werden und das organische Nitrit zudosiert wird.

14. Verfahren nach Anspruch 13, **dadurch gekennzeichnet, dass** jeweils ein Arylamin, ein Olefin und ein organisches Nitrit eingesetzt wird.

15. Verfahren nach einem oder mehreren der Ansprüche 13 bis 14, **dadurch gekennzeichnet, dass** als Arylamin 4-Methoxy-aminobenzol eingesetzt wird.

16. Verfahren nach einem oder mehreren der Ansprüche 13 bis 15, **dadurch gekennzeichnet, dass** als Alkohol 2-Ethyl-hexanol eingesetzt wird.

17. Verfahren nach einem oder mehreren der Ansprüche 13 bis 16, **dadurch gekennzeichnet, dass** als Olefin Acrylsäure eingesetzt wird.

18. Verfahren nach einem oder mehreren der Ansprüche 13 bis 17, **dadurch gekennzeichnet, dass** ein oder mehrere organische Lösungsmittel zugesetzt werden.

19. Verfahren nach einem oder mehreren der Ansprüche 13 bis 18, **dadurch gekennzeichnet, dass** als Säure konzentrierte Schwefelsäure verwendet wird.

## Claims

1. Process for preparing arylolefins by palladium-catalysed coupling of aryldiazonium salts and olefins, **characterized in that**
- one or more arylamines are reacted
- with one or more organic nitrites,
- in the presence of one or more olefins which bear at least one hydrogen atom on the double bond and
- in the presence of acid and
- in the presence of palladium or one or more palladium compounds or palladium and one or more palladium compounds, the olefin, the arylamine, the acid and the palladium catalyst being placed in a reaction vessel and the organic nitrite being added.

2. Process according to Claim 1, **characterized in that** one arylamine is reacted with one organic nitrite in the presence of an olefin which bears at least one hydrogen atom on the double bond and in the presence of palladium or one palladium compound.

3. Process according to Claim 1 or 2, **characterized in that** one or more organic solvents are added.

4. Process according to one or more of Claims 1 to 3, **characterized in that** it is carried out in the presence of one or more free-radical scavengers.

5. Process according to one or more of Claims 1 to 4, **characterized in that** it is carried out in the presence of one or more reducing agents.

6. Process according to one or more of Claims 1 to 5, **characterized in that** the arylamines used are carbocyclic aromatic or heteroaromatic amines.

7. Process according to one or more of Claims 1 to 6, **characterized in that** the arylamines used have the formula (I)
Ar-(NH₂)ₙ (I)
where
n is one or two and
Ar is a carbocyclic aromatic radical having from 6 to 18 framework carbon atoms or a heteroaromatic radical having from 5 to 18 framework carbon atoms in which no, one, two or three framework carbon atoms per ring, but at least one framework carbon atom in the total molecule, is replaced by heteroatoms selected from the group consisting of nitrogen, sulphur and oxygen and the carbocyclic aromatic radicals or heteroaromatic radicals is furthermore substituted by up to five identical or different substituents per ring which are selected from the group consisting of OH, iodine, bromine, chlorine, fluorine, nitro, cyano, free or protected formyl, C₁-C₁₂-alkyl, C₆-C₁₂-aryl, C₇-C₁₃-arylalkyl, C₁-C₈₋hydroxyalkyl, C₁-C₈-hydroxyalkoxy, C₁-C₈-hydroxyalkylamino, -PO-[(C₁-C₈)-alkyl]₂, -PO-[(C₆-C₁₂)-aryl]₂, tri(C₁-C₆-alkyl)siloxyl and radicals of the formula (II),
A-B-D-E (II)
where, independently of one another,
A is absent or is a C₁-C₈-alkylene radical and
B is absent or is oxygen, sulphur or NR¹,
where R¹ is hydrogen, C₁-C₈-alkyl, C₇-C₁₀-arylalkyl or C₆-C₁₀-aryl and
D is a carbonyl group and
E is R², OR², NHR³ or N(R³)₂,
where R² is C₁-C₈-alkyl, C₇-C₁₀-arylalkyl, C₁-C₈-hydroxyalkyl, C₁-C₈₋haloalkyl or C₆-C₁₀-aryl and
R³ are each, independently of one another, C₁-C₈-alkyl, C₁-C₈₋hydroxyalkyl, C₇-C₁₀-arylalkyl or C₆-C₁₀-aryl or
N(R³)₂ together forms a cyclic amino radical,
and radicals of the formulae (IIIa-e)
A-E (IIIa)
A-SO₂-E (IIIb)
A-B-SO₂R² (IIIc)
A-SO₃X (IIId)
A-COX (IIIe)
where A, B, E and R² are as defined above and X is OH, NH₂, or OM, where M is an alkali metal ion, half an equivalent of an alkaline earth metal ion, an ammonium ion or an organic ammonium ion.

8. Process according to one or more of Claims 1 to 7, **characterized in that** the olefins bearing at least one hydrogen atom on the double bond which are used have the formula (V),
R⁵CH=CR⁶R⁷ (V)
where, independently of one another,
R⁵ is hydrogen or methyl and
R⁶ is hydrogen or methyl and
R⁷ is hydrogen, cyano, SO₃M, C₁-C₈-alkyl, a carbocyclic aromatic radical having from 6 to 18 framework carbon atoms or a heteroaromatic radical having from 5 to 18 framework carbon atoms in which no, one, two or three framework carbon atoms per ring, but at least one framework carbon atom in the total molecule, is replaced by heteroatoms selected from the group consisting of nitrogen, sulphur and oxygen
or is a radical of the formula (VI) 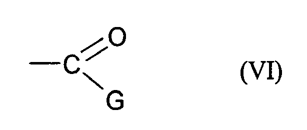 where G is OM, OH, NH₂, OR⁸, NHR⁸ or N(R⁸)₂ and R⁸ is C₁-C₁₂-alkyl, C₇-C₁₂-arylalkyl or C₆-C₁₀-aryl or N(R⁸)₂ together is a cyclic amino radical and M is an alkali metal ion, half an equivalent of an alkaline earth metal ion, an ammonium ion or an organic ammonium ion and
the carbocyclic aromatic radicals or heteroaromatic radicals are further substituted by up to three identical or different substituents per ring which are selected from the group consisting of iodine, bromine, chlorine, fluorine, nitro, cyano, free or protected formyl, C₁-C₁₂-alkyl, C₆-C₁₂-aryl, C₇-C₁₃-arylalkyl, C₁-C₈-alkoxy, C₁-C₈-alkoxycarbonyl, C₁-C₈-haloalkoxy, NH(C₁-C₈-alkyl), N(C₁-C₈-alkyl)₂.

9. Process according to one or more of Claims 1 to 8, **characterized in that** the acid used is sulphuric acid, hydrochloric acid or acetic acid.

10. Process according to one or more of Claims 1 to 9, **characterized in that** the organic nitrites used have the formula (IV),
R⁴ONO (IV)
where
R⁴ is, for example, straight-chain or branched, cyclic or acyclic C₁-C₈₋alkyl or C₇-C₉-arylalkyl, or mixtures of such organic nitrites.

11. Process according to one or more of Claims 1 to 7, **characterized in that** the amount of palladium used or the palladium compound used is from 0.0001 to 10 mol% based on the aryl compound used.

12. Process according to one or more of Claims 1 to 11, **characterized in that** the reaction temperature is from 0 to 150°C.

13. Process according to Claim 1 for preparing cinnamic acid derivatives by palladium-catalysed coupling of aryldiazonium salts and olefins, **characterized in that**
- one or more arylamines are reacted
- with one or more organic nitrites
- in the presence of one or more olefins of the formula (V), where
R⁵ is hydrogen or methyl and
R⁶ is hydrogen or methyl and
R⁷ is a radical of the formula (VI), 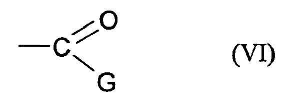
where G is OM, OH, OR⁸, where R⁸ can be C₁-C₁₂-alkyl, C₇-C₁₂₋arylalkyl or C₆-C₁₀-aryl and M is an alkali metal ion, half an equivalent of an alkaline earth metal ion, an ammonium ion or an organic ammonium ion
- in the presence of acid and
- in the presence of palladium or one or more palladium compounds or palladium and one or more palladium compounds and
- in the presence of an alcohol of the formula (XI)
R¹³OH (XI)
- where R¹³ is C₁-C₁₂-alkyl or C₇-C₁₃-arylalkyl, the olefin, the arylamine, the acid and the palladium catalyst being placed in a reaction vessel and the organic nitrite being added.

14. Process according to Claim 13, **characterized in that** in each case one arylamine, one olefin and one organic nitrite are used.

15. Process according to Claim 13 or 14, **characterized in that** 4-methoxy-aminobenzene is used as arylamine.

16. Process according to one or more of Claims 13 to 15, **characterized in that** 2-ethylhexanol is used as alcohol.

17. Process according to one or more of Claims 13 to 16, **characterized in that** acrylic acid is used as olefin.

18. Process according to one or more of Claims 13 to 17, **characterized in that** one or more organic solvents are added.

19. Process according to one or more of Claims 13 to 18, **characterized in that** concentrated sulphuric acid is used as acid.

## Revendications

1. Procédé de préparation d'aryloléfines par couplage catalysé au palladium de sels d'aryldiazonium et d'oléfines, **caractérisé en ce que**
on fait réagir
- une ou plusieurs arylamines
- en présence d'une ou plusieurs oléfines, qui portent au moins un atome d'hydrogène sur la double liaison et
- en présence d'un acide et
- en présence de palladium ou d'un ou de plusieurs composés du palladium, ou du palladium et un ou plusieurs composés du palladium
- avec un ou plusieurs nitrites organiques,
où l'oléfine, l'arylamine, l'acide et le catalyseur au palladium sont introduits initialement et le nitrite organique est ensuite ajouté en quantité mesurée.

2. Procédé selon la revendication 1, **caractérisé en ce qu'**une arylamine est mise à réagir avec un nitrite organique en présence d'une oléfine, qui porte au moins un atome d'hydrogène sur la double liaison, en présence de palladium ou d'un composé du palladium.

3. Procédé selon l'une ou plusieurs des revendications 1 à 2, **caractérisé en ce que** l'on ajoute un ou plusieurs solvants organiques.

4. Procédé selon l'une ou plusieurs des revendications 1 à 3, **caractérisé en ce qu'**il est réalisé en présence d'un ou de plusieurs capteurs de radicaux.

5. Procédé selon l'une ou plusieurs des revendications 1 à 4, **caractérisé en ce qu'**il est réalisé en présence d'un ou de plusieurs réducteurs.

6. Procédé selon l'une ou plusieurs des revendications 1 à 5, **caractérisé en ce que**, en tant qu'arylamines, on utilise des amines carbocycliques aromatiques ou hétéroaromatiques.

7. Procédé selon l'une ou plusieurs des revendications 1 à 6, **caractérisé en ce que**, en tant qu'arylamines, on utilise celles de formule générale (I)
Ar-(NH₂)ₙ (I)
dans laquelle
n vaut un ou deux et
Ar représente des radicaux carbocycliques aromatiques renfermant de 6 à 18 atomes de carbone du squelette, ou des radicaux hétéroaromatiques renfermant de 5 à 18 atomes de carbone du squelette, dans lesquels zéro, un, deux ou trois atomes de carbone du squelette par cycle, mais au moins un atome de carbone du squelette dans l'ensemble de la molécule, est (sont) substitué(s) par des hétéroatomes choisis parmi le groupe constitué d'un atome d'azote, d'un atome de soufre ou d'un atome d'oxygène, et les radicaux carbocycliques aromatiques ou les radicaux hétéroaromatiques sont en outre substitués par jusqu'à cinq substituants identiques ou différents par cycle, choisis parmi le groupe constitué d'un groupe OH, d'un atome d'iode, d'un atome de brome, d'un atome de chlore, d'un atome de fluor, d'un groupe nitro, d'un groupe cyano, d'un groupe formyle libre ou protégé, d'un groupe alkyle en C₁-C₁₂, d'un groupe aryle en C₆-C₁₂, d'un groupe arylalkyle en C₇-C₁₃, d'un groupe hydroxyalkyle en C₁-C₈, d'un groupe hydroxyalcoxy en C₁-C₈, d'un groupe hydroxyalkylamino en C₁-C₈, d'un groupe -PO-[(C₁-C₈) alkyle]₂, d'un groupe -PO- [(C₆-C₁₂)-aryle]₂, d'un groupe tri (C₁-C₆)alkyl)siloxyle ou de radicaux de formule générale (II),
A-B-D-E (II)
dans laquelle indépendamment les uns des autres,
A est absent ou représente un radical alkylène en C₁-C₈ et
B est absent ou représente un atome d'hydrogène, un atome de soufre ou un groupe NR¹,
dans lequel R¹ représente un atome d'hydrogène, un groupe alkyle en C₁-C₈, un groupe arylalkyle en C₇₋C₁₀, ou un groupe aryle en C₆-C₁₀ et
D représente un groupe carbonyle et
E représente un groupe R², un groupe OR², un groupe NHR³ ou un groupe N(R³)₂,
dans lesquels R² représente un groupe alkyle en C₁-C₈, un groupe arylalkyle en C₇-C₁₀, un groupe hydroxyalkyle en C₁-C₈, un groupe halogénoalkyle en C₁-C₈, ou un groupe aryle en C₆-C₁₀, et
R³ représente chacun indépendamment un groupe alkyle en C₁-C₈, un groupe hydroxyalkyle en C₁-C₈, un groupe arylalkyle en C₇-C₁₀, ou un groupe aryle en C₆-C₁₀, ou
N(R³)₂ représente conjointement un radical amino cyclique
ou des radicaux de formules générales (IIIa-e)
A-E (IIIa)
A-SO₂-E (IIIb)
A-B-SO₂R² (IIIc)
A-SO₃X (IIId)
A-COX (IIIe)
dans lesquelles A, B, E et R² présentent la signification indiquée ci-dessus et X représente un groupe OH, un groupe NH₂, ou un groupe OM, dans lequel M représente un ion de métal alcalin, un demi-équivalent d'un ion de métal alcalino-terreux, un ion ammonium ou un ion ammonium organique.

8. Procédé selon l'une ou plusieurs des revendications 1 à 7, **caractérisé en ce que**, en tant qu'oléfines qui portent au moins un atome d'hydrogène sur la double liaison, on utilise celles de formule générale (V),
R⁵CH=CR⁶R⁷ (V)
dans laquelle, indépendamment les uns des autres,
R⁵ représente un atome d'hydrogène ou un groupe méthyle, et
R⁶ représente un atome d'hydrogène ou un groupe méthyle, et
R⁷ représente un atome d'hydrogène, un groupe cyano, un groupe SO₃M, un groupe alkyle en C₁₋C₈, des radicaux carbocycliques aromatiques renfermant de 6 à 18 atomes de carbone du squelette ou des radicaux hétéroaromatiques renfermant de 5 à 18 atomes de carbone du squelette, dans lesquels zéro, un, deux ou trois atomes de carbone du squelette par cycle, mais au moins un atome de carbone du squelette dans l'ensemble de la molécule, est (sont) substitué(s) par des hétéroatomes choisis parmi le groupe constitué d'un atome d'azote, d'un atome de soufre ou d'un atome d'oxygène,
ou des radicaux de formule générale (VI) 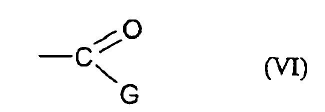 dans laquelle G représente un groupe OM, un groupe OH, un groupe NH₂, un groupe OR⁸, un groupe NHR⁸ ou un groupe N(R⁸)₂, et R⁸ représente un groupe alkyle en C₁-C₁₂, un groupe arylalkyle en C₇-C₁₂, ou un groupe aryle en C₆-C₁₀, ou
N(R⁸)₂ représente conjointement un radical amino cyclique, et où M représente un ion de métal alcalin, un demi-équivalent d'un ion de métal alcalino-terreux, un ion ammonium ou un ion ammonium organique, et
en outre, les radicaux carbocycliques aromatiques ou les radicaux hétéroaromatiques sont substitués par jusqu'à trois substituants identiques ou différents par cycle, choisis parmi le groupe constitué d'un atome d'iode, d'un atome de brome, d'un atome de chlore, d'un atome de fluor, d'un groupe nitro, d'un groupe cyano, d'un groupe formyle libre ou protégé, d'un groupe alkyle en C₁₋C₁₂, d'un groupe aryle en C₆-C₁₂, d'un groupe arylalkyle en C₇-C₁₃, d'un groupe alcoxy en C₁-C₈, d'un groupe C₁-C₈-alcoxycarbonyle, d'un groupe halogénoalcoxy en C₁-C₈, d'un groupe NH (C₁-C₈₋alkyle) et d'un groupe N(C₁-C₈-alkyle)₂.

9. Procédé selon l'une ou plusieurs des revendications 1 à 8, **caractérisé en ce que**, en tant qu'acides, on utilise l'acide sulfurique, l'acide chlorhydrique ou l'acide acétique.

10. Procédé selon l'une ou plusieurs des revendications 1 à 9, **caractérisé en ce que**, en tant que nitrites organiques, on utilise ceux de formule générale (IV),
R⁴ONO (IV)
dans laquelle
R⁴ représente par exemple, un groupe alkyle en C₁₋C₈ linéaire ou ramifié, cyclique ou acyclique, ou un groupe arylalkyle en C₇-C₉, ou des mélanges de tels nitrites organiques.

11. Procédé selon l'une ou plusieurs des revendications 1 à 7, **caractérisé en ce que** la quantité du palladium utilisé ou du composé du palladium utilisé est de 0,0001 à 10 % en moles, par rapport au composé arylique utilisé.

12. Procédé selon l'une ou plusieurs des revendications 1 à 11, **caractérisé en ce que** la température de réaction est de 0 à 150°C.

13. Procédé selon la revendication 1 pour la préparation de dérivés de l'acide cinnamique par couplage catalysé au palladium de sels d'aryldiazonium et d'oléfines, **caractérisé en ce que**
on fait réagir
- une ou plusieurs arylamines
- en présence d'une ou de plusieurs oléfines de formule générale (V), dans laquelle
R⁵ représente un atome d'hydrogène ou un groupe méthyle et
R⁶ représente un atome d'hydrogène ou un groupe méthyle et
R⁷ représente des radicaux de formule générale (VI) 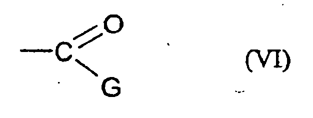 dans laquelle G peut représenter un groupe OM, un groupe OH, un groupe OR⁸, et R⁸ peut représenter un groupe alkyle en C₁-C₁₂, un groupe arylalkyle en C₇-C₁₂, ou un groupe aryle en C₆-C₁₀, et où M représente un ion de métal alcalin, un demi-équivalent d'un ion de métal alcalino-terreux, un ion ammonium ou un ion ammonium organique, et
- en présence d'un acide et
- en présence de palladium ou d'un ou de plusieurs composés du palladium,
ou du palladium ou un ou plusieurs composés du palladium,
- en présence d'un alcool de formule générale (XI)
R¹³OH (XI)
- dans laquelle R¹³ représente un groupe alkyle en C₁-C₁₂ ou un groupe arylalkyle en C₇-C₁₃
- avec un ou plusieurs nitrites organiques,
où l'oléfine, l'arylamine, l'acide et le catalyseur au palladium sont introduits initialement et le nitrite organique est ajouté en quantité mesurée.

14. Procédé selon la revendication 13, **caractérisé en ce que** l'on utilise dans chaque cas une arylamine, une oléfine et un nitrite organique.

15. Procédé selon l'une ou plusieurs des revendications 13 à 14, **caractérisé en ce que**, en tant qu'arylamine, on utilise le 4-méthoxy-aminobenzène.

16. Procédé selon l'une ou plusieurs des revendications 13 à 15, **caractérisé en ce que**, en tant qu'alcool, on utilise le 2-éthyl-hexanol.

17. Procédé selon l'une ou plusieurs des revendications 13 à 16, **caractérisé en ce que**, en tant qu'oléfine, on utilise l'acide acrylique.

18. Procédé selon l'une ou plusieurs des revendications 13 à 17, **caractérisé en ce que** l'on ajoute un ou plusieurs solvants organiques.

19. Procédé selon l'une ou plusieurs des revendications 13 à 18, **caractérisé en ce que**, en tant qu'acide, on utilise de l'acide sulfurique concentré.
